(19) Europäisches Patentamt
European Patent Office
Office européen des brevets

(11) Publication number: 0 162 695
B1

(12) **EUROPEAN PATENT SPECIFICATION**

(45) Date of publication of patent specification: 17.01.90

(51) Int. Cl.$^5$: C 07 D 209/90

(21) Application number: 85303556.6

(22) Date of filing: 21.05.85

# (54) 6-Oxygenated-1,3,4,5-Tetrahydrobenz(cd)indol-4-amines.

(30) Priority: 24.05.84 US 613839

(43) Date of publication of application:
27.11.85 Bulletin 85/48

(45) Publication of the grant of the patent:
17.01.90 Bulletin 90/03

(84) Designated Contracting States:
AT BE CH DE FR GB IT LI LU NL SE

(56) References cited:
EP-A-0 002 570
EP-A-0 029 581
EP-A-0 148 440
EP-A-0 153 083
CH-A- 517 732
US-A-3 336 307

CHEMICAL ABSTRACTS, vol. 102, 1985, page 18, no. 142802m, Columbus, Ohio, US; A.P. MAZUREK et al.: "Theoretical and experimental studies of drug-receptor interactions: determinants for recognition of 5-hydroxytryptamine analogs" & INT. J. QUANTUM CHEM., QUANTUM BIOL. SYMP. 1984, 11, 183-94

(73) Proprietor: SMITHKLINE BEECHAM CORPORATION
P.O. Box 7929 1 Franklin Plaza
Philadelphia Pennsylvania 19101 (US)

(72) Inventor: Kaiser, Carl
1105 Sylvan Drive
Haddon Heights New Jersey 08035 (US)
Inventor: Kruse, Lawrence Ivan
646 Clinton Avenue
Haddonfield New Jersey 08033 (US)

(74) Representative: Waters, David Martin, Dr. et al
Smith Kline & French Laboratories Ltd. Patent Department Mundells
Welwyn Garden City Hertfordshire AL7 1EY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 162 695 B1

## Description

This invention relates to new 6-oxygenated-1,3,4,5-tetrahydrobenz[cd]indol-4-amines, to pharmaceutical compositions containing these compounds and to the use of the compounds in preparing a composition for modulating serotoneric activity. The invention also relates to a process for preparing these compounds and to indol-4-one intermediates. Certain 1,3,4,5-tetrahydrobenz[cd]indol-4-amines are known in the prior art. EP—148440—A discloses such compounds in which the 6-position is substituted by a methoxy group and the 4-position amine is a di-$C_{1-4}$alkylamine or diallylamine; and EP—153083—A discloses such compounds in which (i) the 6-position is substituted by a hydroxy group and the 4-position amine is unsubstituted, a dipropylamine group, a diallylamine group, or a methylethylamine group, (ii) the 6-position is substituted by an ethoxy group and the 4-position amine is a diallylamine, and (iii) the 6-position is substituted by a propoxy group and the 4-position amine is a dimethylamine group. Both documents are prior art under Article 54 (3) EPC.

The compounds of this invention are selective serotonin (5—HT) receptor antagonists. They bind selectively to 5—$HT_1$ (vs 5—$HT_2$) serotonin receptors. Such selective 5—$HT_1$ receptor antagonists are useful, for example, in cardiovascular, gastrointestinal and renal disease states, as well as in the treatment of other disorders, such as depression, in which serotonin modulation is important. The compounds have antihypertensive activity.

The 6-oxygenated-1,3,4,5-tetrahydrobenz[cd]indol-4-amine compounds of this invention are represented by the following formula (I):

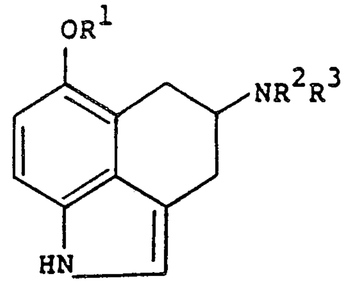

(I)

in which:

$R^1$ is hydrogen or $C_{1-5}$ alkyl; and

$R^2$ and $R^3$, being the same or different, are hydrogen, $C_{1-4}$ alkyl, phenyl $(CH_2)_n$ where n is 0—3, $C_{5-6}$ cycloalkyl or $C_{3-5}$ alkenyl;

or a pharmaceutically acceptable acid addition salt thereof, provided that, when

i) $R^1$ is $C_{1-4}$alkyl, $R^2$ and $R^3$ are not both $C_{1-4}$alkyl or allyl, or pharmaceutically acceptable salts thereof except the oxalate salt of the compound in which $R^1$ to $R^3$ are all methyl;

ii) $R^1$ is hydrogen, $R^2$ and $R^3$ are not both $C_{1-4}$alkyl or allyl, or pharmaceutically acceptable salts thereof.

Particular compounds of formula (I) are those in which $R^1$ is hydrogen or methyl.

Other particular compounds of formula (I) are those in which $R^2$ and $R^3$ are both hydrogen or are both methyl.

Specific compounds of formula (I) are:

6-methoxy-1,3,4,5-tetrahydrobenz[cd]indol-4-amine

N,N-dimethyl-6-methoxy-1,3,4,5-tetrahydrobenz[cd]indol-4-amine oxalate.

The compounds of formula (I) are prepared by the following procedures.

Method A

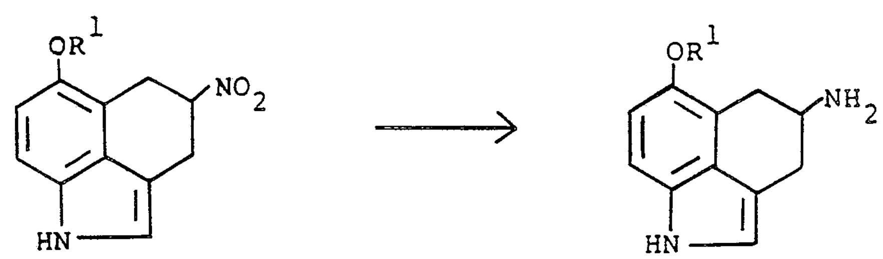

(II)

$R^1$ is defined as in formula (I).

According to the procedure of Method A, a 4-nitro-1,3,4,5-tetrahydrobenz[cd]indole (II) is hydrogenated using a noble metal catalyst such as palladium, or preferably, platinum oxide.

Method B

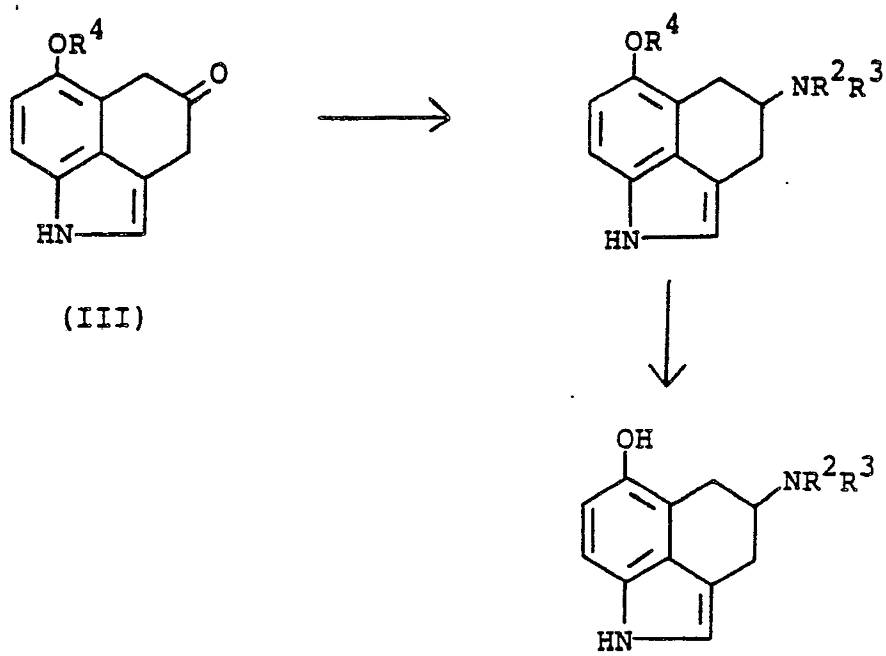

(III)

$R^4$ is $C_{1-5}$ alkyl and $R^2$ and $R^3$ are as defined in formula (I).

The first step in Method B, which is a reductive amination method, is also included in this invention. The reductive amination is carried out with sodium cyanoborohydride and an amine of the formula:

$$R^2R^3NH$$

and an inorganic acid addition salt, such as a hydrochloride salt, of said amine. The process is preferably carried out with the inorganic acid addition salt of the amine and the basic amine present in a ratio of about 10:1 to 10:3.

According to the second step of Method B, compounds of formula (I) where $R^1$ is hydrogen are prepared by dealkylating the 6-alkoxy compounds using, for example, boron tribromide, hydrobromic acid or pyridine hydrochloride.

The compounds of formula (I) where $R^2$ and $R^3$ are other than hydrogen may be prepared by alkylation of the unsubstituted amino compounds when $R^1$ is $C_{1-5}$ alkyl. However, these compounds, particularly where $R^2$ and $R^3$ are both other than hydrogen, are preferably prepared by the reductive amination process of Method B.

The secondary amine compounds of formula (I), i.e. where only one of $R^2$ or $R^3$ is other than hydrogen, may be prepared by acylating the primary amine with an acylating agent such as an acid halide, anhydride or activated ester such as a p-nitrophenyl or dinitrophenyl ester, and then reducing, for example with an alkali metal hydride such as lithium (or sodium) aluminum hydride, or other reducing agent such as borane or borane/tetrahydrofuran or borane/dimethylsulfide, to give the N-substituted amines of formula (I).

The compounds of formula (I) form pharmaceutically acceptable acid addition salts with organic or inorganic acids. Examples of these acids are hydrochloric, hydrobromic, sulfuric, phosphoric, acetic, tartaric, citric, maleic, lactic, oxalic, succinic, methanesulfonic, and benzenesulfonic acids. The salts are formed according to methods known in the art. If the product is isolated as acid addition salt, it may be treated with base such as aqueous sodium hydroxide and converted to the corresponding base. The base can then be treated with an appropriate acid for example in an aqueous miscible solvent, such as a lower alkanol preferably methanol or ethanol, to give the desired salt.

The indol-4-one intermediates of this invention are represented by the following formula (IV):

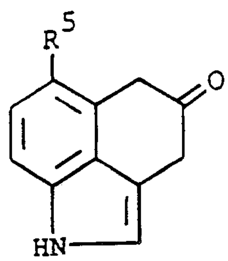

(IV)

in which $R^5$ is $C_{1-5}$ alkoxy.

The compounds of formula (IV) in which $R^5$ is $C_{1-5}$ alkoxy are intermediates of formula (III) in Method B. It is also to be noted that, although not claimed herein, those compounds of formula (IV) in which $R^5$ is hydrogen are intermediates, for example, for the preparation of the N,N-dipropyl-1,3,4,5-tetrahydrobenz[cd]indol-4-amine of U.S. 4,110,339 by reductive amination.

The 4-nitro-tetrahydrobenz[cd] indole intermediates of formula (II) in Method A are prepared by the following process.

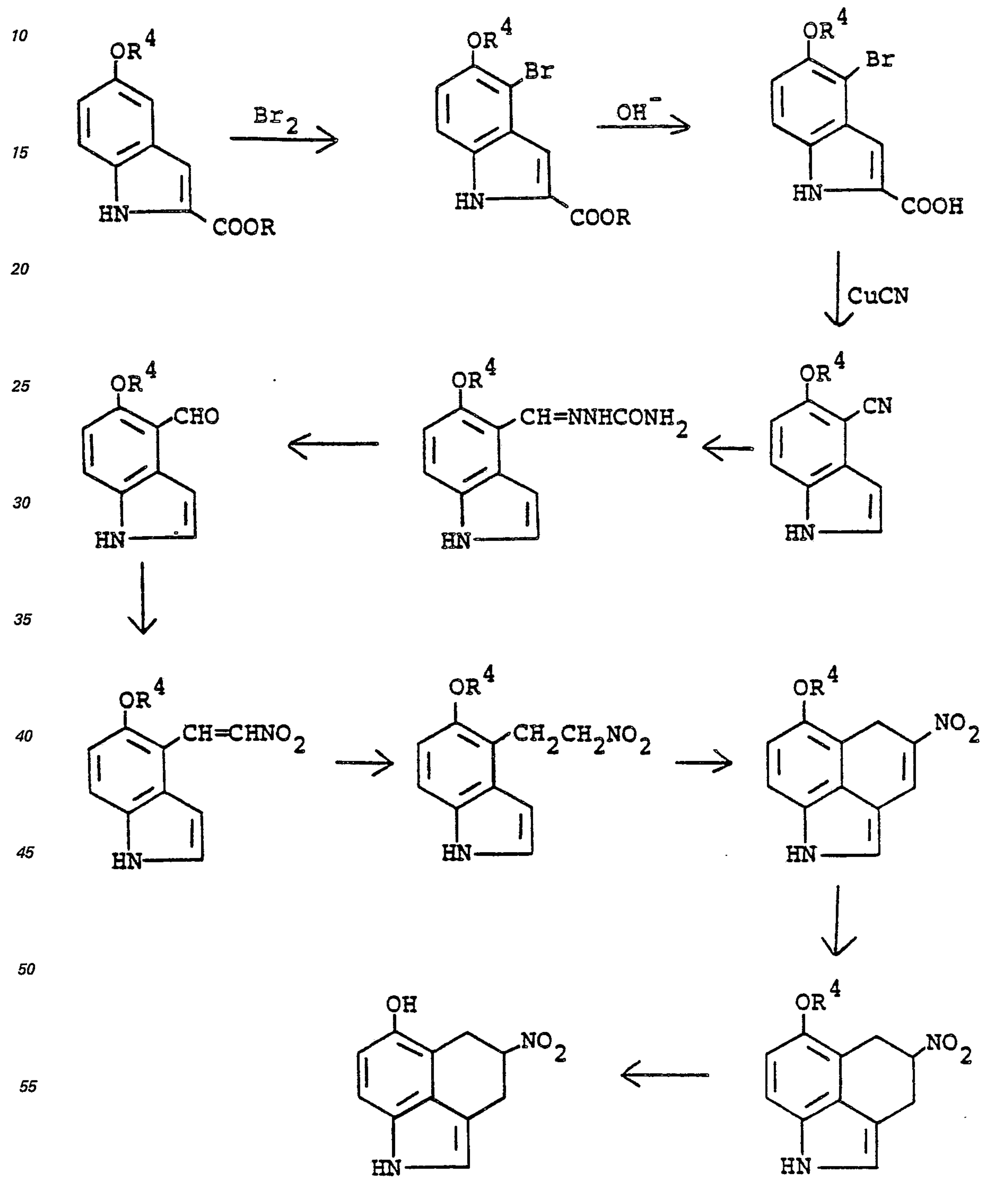

R is methyl or ethyl and $R^4$ is $C_{1-5}$ alkyl. This process is illustrated in examples 1 and 2.

The 4-keto intermediates of formula (III) are prepared by treating the 4-nitro compounds (formula II where $R^1$ is $C_{1-5}$ alkyl) with solid sodium methoxide in methanol, then with aqueous titanium trichloride. This process is illustrated in example 3 herebelow.

The selective 5—$HT_1$ receptor antagonist activity of the compounds of formula (I) is demonstrated by the displacement of [$^3$H]5—HT from rat frontal cerebral cortex preferably over the displacement of [$^3$H]spiroperidol which binds preferentially with 5—$HT_2$ receptors. Peroutka et al, *Molecular Pharmacology* 16:687—699 (1979) which describes this test procedure and designate as 5—$HT_1$ and 5—$HT_2$ those serotonin receptors that are labeled by [$^3$H]5-HT and [$^3$H]spiroperidol, respectively. The $IC_{50}$ (concentration required to displace 50% of [$^3$H]5—HT, binding from rat frontal cerebral cortex) for compounds of examples 1—3 is about 40—70nM.

Also, selective 5—$HT_1$ receptor antagonist activity of the compounds of formula (I) may be shown by the ability to antagonize the effect of serotonin in an in vitro dog basilar artery preparation, Peroutka et al, *Brain Research* 259:327—330 (1983). The compound of example 1 herebelow showed selective 5—$HT_1$ activity at $K_B = 4.63 \times 10^{-7}$M.

The inhibition of the activity of serotonin (i.e. 5-hydroxytryptamine) is an activity known to the art; in U.S. 4,435,405, certain quinoline compounds are described as 5-hydroxytryptamine antagonists which demonstrated binding at 5—$HT_1$ and 5—$HT_2$ receptors.

Antihypertensive activity of compounds of formula (I) is demonstrated by administration to spontaneously hypertensive rats (SHR) at doses of about 0.4—1.0 mg/kg, i.v. In this test procedure, diastolic blood pressure and heart rate are reduced.

This invention also includes pharmaceutical compositions having 5—$HT_1$ receptor antagonist activity comprising a compound of formula (I) or a pharmaceutically acceptable acid addition salt thereof and a pharmaceutically acceptable carrier.

The pharmacologically active compounds of formula (I) can be administered orally or parenterally. Preferably, these compounds are administered in conventional dosage unit forms, prepared by combining an appropirate dose of the compound with standard pharmaceutical carriers. The dosage units will contain the active ingredient in an effective amount selected from about 0.01 mg. to about 500 mg., preferably 1 mg. to 50 mg.

The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the like. Similarly, the carrier of diluent can include any time delay material, well known to the art, such as glyceryl monostearate or glyceryl distearate alone or with a wax.

A wide variety of pharmaceutical forms can be employed. Thus, if a solid carrier is used the preparation can be tableted, placed in hard gelatin capsule in powder or pellet form or in the form of a trouche or lozenge. The amount of solid carrier will vary widely but preferably will be from about 25 mg. to about 1 g. If a liquid carrier is used, the preparation will be in the form of a syrup, emulsion, soft gelatin capsule, sterile injectable liquid such as an ampul or an aqueous or nonaqueous liquid suspension.

The pharmaceutical compositions are prepared by conventional techniques involving procedures such as mixing, granulating and compressing when necessary or variously mixing and dissolving the ingredients as appropriate to the desired composition.

The use of the compounds in the preparation of compositions for inhibiting the action of serotonin at the 5—$HT_1$ receptors in accordance with this invention comprises administering internally to a subject in need of said activity a compound of formula (I) or a pharmaceutically acceptable acid addition salt thereof, in an amount sufficient to produce said activity.

Also, included in this invention is the use of the compounds in the preparation of antihypertensive active compositions for administering internally to a subject in need of said activity a compound of formula (I) or a pharmaceutically acceptable acid addition salt thereof, in an amount sufficient to produce said activity.

The compound will preferably be administered in a dosage unit form orally or parenterally. Advantageously equal doses will be administered one to four times daily with the daily dosage regimen being from about 1 mg. to about 250 mg., preferably from 1 mg. to 10 mg. When the method described above is carried out, the action of serotonin at the 5—$HT_1$ receptors is inhibited.

One skilled in the art will recognise that in determining the amounts of the compound needed to produce the desired pharmacological effect without toxic side effects, the activity of the particular compound as well as the size of the host animal must be considered.

The following examples illustrate the invention but are not to be construed as limiting the scope thereof. Temperatures are in degrees Centigrade unless otherwise stated.

## Example 1

A mixture of ethyl 5-methoxy-(1H)indole-2-carboxylate (186 g, 0.85 mol) and glacial acetic acid (4.2 L) was stirred and warmed on a hot plate until only a small amount of indole remained undissolved. The warm solution was stirred vigorously during the dropwise addition (15 min) of bromine (43.4 mL, 135.4 g, 0.85 mol), then allowed to stand at ambient temperature for 24 hours. The resulting mixture was filtered,

the crystalline product was washed sequentially with acetic acid (2 × 150 mL), hexane ( 2 × 200 mL), and then dried at 65°C to give ethyl 4-bromo-5-methoxy-(1H)indole-2-carboxylate m.p. 176— 178°C (softens at 166°C).

A 5-L flask was charged with sodium hydroxide (60 g, 1.5 mol) and water (0.5 L), and the contents were stirred until homogeneous. Ethanol (1 L) tetrahydrofuran (0.25 L), and ethyl 4-bromo-5-methoxy-(1H)indole-2-carboxylate (180 g, 0.6 mol) were added and the mixture was heated at reflux for 1 hour, then cooled to ambient temperature. The solution was stirred vigorously as concentrated hydrochloric acid (0.25 L, 3 mol) and water (2.5 L) were added. The resulting thick yellow paste was cooled at 0°C overnight, filtered, and the crystalline product washed with water (3 × 400 mL), and dried at 60°C to give 4-bromo-5-methoxy-(1H)indole-2-carboxylic acid (161 g, 99.3%), m.p. 263°C(d).

A 1-L flask was charged with freshly distilled N,N'-dimethylacetamide (450 mL) and the solvent was degassed (15 min) with a vigorous stream of argon. 4-Bromo-5-methoxy-(1H)indole-2-carboxylic acid (75 g, 0.28 mol) and copper (I) cyanide (75 g, 0.84 mole) were added, the solution was stirred and heated at reflux for 28 hours, cooled to ambient temperature, and poured into a mixture of water (500 mL) and ethyl acetate (500 mL). The mixture was filtered through diatomaceous earth, the precipitate was washed with ethyl acetate (250 mL), the combined ethyl acetate filtrates were separated from the aqueous layer, and washed with water (5 × 250 mL) and brine. The solution was dried and concentrated, and the residue was recrystallized from ethyl acetate/hexane to give 5-methoxy-(1H)indole-2-carbonitrile (21 g, 44%), m.p. 139—141°C.

A 450-mL Parr hydrogenation bottle was charged with sodium acetate (12 g , 0.146 mol), semicarbazide hydrochloride (16 g, 0.144 mol) and water (45 mL), and the mixture was heated until homogeneous. A heaping tablespoon of Raney nickel catalyst was added to the cooled Parr bottle followed by a solution of 5-methoxy-(1H)indole-4-carbonitrile (20 g, 0.116 mol) in hot methanol (185 mL). The mixture was hydrogenated at 50 psi until the theoretical uptake of hydrogen was complete (18—24 hours). The mixture was heated to near boiling on a steam bath, filtered hot, and the precipitate was washed with hot N,N-dimethylformamide until the catalyst was free of product. The filtrate was concentrated and traces of solvent were removed from the residue by further concentration using a dry ice cold trap to give a solid residue. The residue was triturated with a mixture of ethanol (60 mL) and water (180 mL), filtered, washed with water and dried at 60°C to yield 5-methoxy-(1H)indole-4-carboxaldehyde semicarbazone (18.89 g, 70%), m.p. 219—221°C(d)

A mixture of water (0.46 L), glacial acetic acid (0.91 L), freshly distilled pyruvic acid (33.23 g, 0.378 mol), and anhydrous sodium acetate (34.16 g, 0.146 mol) was stirred until homogeneous, then 5-methoxy-(1H)indole-4-carboxaldehyde semicarbazone (17.5 g, 0.0754 mol) was added and the mixture was stirred for 16 hours. The resulting solution was concentrated with a dry-ice cold trap (water bath temperature <25°C), the residue was taken up in ethyl acetate (0.5 L) and filtered, and the filtrate was washed sequentially with water (2 × 250 mL), 5% aqueous sodium carbonate (3 × 250 mL), brine, dried, and warmed briefly with decolorizing charcoal before concentration. The crude product was dissolved in a little ethyl acetate and applied to a silica gel (250 g) column; elution with 2:1 ethyl acetate-hexane and concentration of the first 1.0 L of eluate gave 5-methoxy-(1H)indole-4-carboxaldehyde (9.8 g, 74.2%), m.p. 134—136°C (softens at 125°C).

A solution of 5-methoxy-(1H)indole-4-carboxaldehyde (8.61 g, 49.2 mmol) and ammonium acetate (1.5 g) in nitromethane (70 mL) was heated on a steam bath for 3 hours, and diluted with ethyl acetate (150 mL). The solution was washed twice with water, once with brine, dried, and concentrated. The solid product was recrystallized from ethyl acetate/hexane. The crystallization filtrates were concentrated, purified by flash chromatography using 1:1 hexane-ethyl acetate as eluant, recrystallized from ethyl acetate/hexane, and combined with the first crop of product to give 5-methoxy-4-(2-nitroethenyl)-(1H)indole (9.15 g, 85%), m.p. 175—176°C.

A 2-L flask was charged with methanol (0.42 L) and 5-methoxy-4-(2-nitroethenyl)-(1H)indole (9.1 g, 41.7 mmol), and the resulting mixture was stirred during the slow (20 min) addition of sodium borohydride (7.0 g, 0.185 mol). During the addition, vigorous hydrogen evolution occurred and the mixture became warm. The solution was stirred for 20 minutes after the addition was completed, glacial acetic acid (15 mL) was added to a pH of 6, and the solution was concentrated. The solid residue was partitioned between water and ethyl acetate. The aqueous layer was washed twice with ethyl acetate and the combined ethyl acetate phases were washed with water, twice with 10% aqueous sodium carbonate, brine, then dried, and concentrated. The resulting oil was purified by flash chromatography using 40% ethyl acetate-hexane as eluant to give 5-methoxy-4-(2-nitroethyl)-(1H)indole (8.38 g, 91%), m.p. 77—79°C.

N,N-Dimethylformamide (20 mL) was stirred under argon in a 500 mL flask during the addition (over 2 minutes) of phosphorus oxychloride (3.45 mL, 37.1 mmol), then the flask was cooled in a cold water bath during the addition (over 2 minutes) of 5-methoxy-4-(2-nitroethyl)-(1H)indole (7.15 g, 32.5 mmol). After the addition was completed, the cooling bath was removed, stirring was continued for 15 min at ambient temperature, a mixture of water (6.5 mL, 0.36 mol) and N,N-dimethylformamide (26 mL) was added, and the resulting solution was stirred for 10 min. A mixture of triethylamine (45.5 mL, 0.33 mol) and methanol (130 mL) was added, the solution was heated at reflux for 30 min, water (75 mL) was added slowly to the hot solution and the mixture was cooled at 20°C for 2 hours. The mixture was filtered and the precipitate was washed sequentially with water, 2:1 methanol-water (2 × 25 mL), ether (3 × 50 mL), and then dried

at 60°C to yield a 6-methoxy-4-nitro-1,5-dihydrobenz[cd]indole (6.8 g, 91%), m.p. 350°C(d).

A mixture of 6-methoxy-4-nitro-1,5-dihydrobenz[cd]indole (7.26 g, 31.6 mmol) and methanol (0.5 L) was stirred vigorously in a 2-L flask during the slow (20 min) addition of sodium borohydride (18.25 g, 0.48 mol). During the addition, vigorous hydrogen evolution occurred and the mixture became quite warm. After stirring an additional 10 min, glacial acetic acid (45 mL) was added to a final pH of 5—6. The solution was concentrated, partitioned between water and ethyl acetate, the aqueous layer was extracted twice with ethyl acetate, and the combined organic layers were washed sequentially with water, 5% aqueous sodium carbonate, and brine. The solution was dried and concentrated, and the solid residue was purified by flash chromatography using 2:1 hexane-ethyl acetate as eluant to give 6-methoxy-4-nitro-1,3,4,5-tetrahydrobenz[cd]indole (6.5 g, 89%), m.p. 134—136°C.

Platinum oxide (300 mg) was reduced under 60 psi hydrogen pressure in methanol (30 mL) for 30 min. 6-Methoxy-4-nitro-1,3,4,5-tetrahydrobenz[cd]indole (10 g, 4.31 mmol) was added and the resulting solution was hydrogenated under 60 psi hydrogen pressure until hydrogen uptake ceased (about 2 hours). The mixture was filtered, the filtrate was concentrated, and the residue was dissolved in chloroform and decolorized with activated carbon. The resulting mixture was filtered and the filtrate was concentrated. The residue, which contained 6-methoxy-4-nitro-1,3,4,5-tetrahydrobenz[cd]indole-4-amine, was dissolved in absolute ethanol (8 mL) and the solution was stirred and heated to near reflux as a solution of oxalic acid dihydrate (540 mg, 4.31 mmol) in absolute ethanol (5 mL) was added slowly. The mixture was diluted with acetone (10 mL), cooled at 0°C, filtered, and the precipitate was washed with acetone (2 × 10 mL), hot methanol (2 × 10 mL), and dried to yield 6-methoxy-1,3,4,5-tetrahydrobenz[cd]indole-4-amine hemioxalate (0.74 g, 70%), m.p. 260—265°C(d).

Example 2

6-Methoxy-4-nitro-1,3,4,5-tetrahydrobenz[cd]indole (0.210 g) was dissolved in 30 ml of anhydrous methylene chloride under nitrogen in a flame dried flask. The mixture was cooled to 0°C and 4 mL of 1 molar boron tribromide in methylene chloride was added. The mixture was stirred at 0°C for 6 hours. The reaction was quenched with 5% aqueous sodium bicarbonate and extracted twice with ethyl acetate. The organic extracts were dried and the solvent removed. The residue was chromatographed over silica using 60/40 hexane/ethyl acetate to give 6-hydroxy-4-nitro-1,3,4,5-tetrahydrobenz[cd]indole.

6-Hydroxy-4-nitro-1,3,4,5-tetrahydrobenz[cd]indole (52 mg) was dissolved in 10 mL of methanol. The solution was added to 50 mg of platinum oxide which had been reduced with hydrogen at 50 psi in 10 mL of methanol for 3 hours, then filtered under argon into a flask containing 21.5 mg (1 equiv.) of oxalic acid in 2 mL of methanol. The solution was concentrated to about 10 mL volume and 100 mL of diethyl ether was added. After 1 hour at −20°C, the solid material was filtered, washed with diethyl ether and dried to yield 31 mg of 6-hydroxy-1,3,4,5-tetrahydrobenz[cd]indole-4-amine oxalate, m.p. 218—220°C(d).

Example 3

A mixture of 6-methoxy-1,3,4,5-tetrahydrobenz[cd]indole (1.16 g, 5 mmol) and methanol (25 mL) was treated with solid sodium methoxide (0.3 g, 5.5 mol) and stirred under argon until homogeneous. A solution made by mixing 20% aqueous titanium trichloride (17.5 mL) with ammonium acetate (10.5 g) dissolved in water (35 mL) was added, and the resulting suspension was stirred for 1 hour. The mixture was shaken vigorously and decanted with diethyl ether (8 × 50 mL). The diethyl ether extracts were washed sequentially with water (3 × 100 mL), 5% aqueous sodium carbonate (2 × 100 mL) and brine, then dried and concentrated to give 6-methoxy-1,5-dihydrobenz[cd]indol-4(3H)-one (0.82 g, 81%) m.p. 130—132°C(d).

A solution of anhydrous dimethylamine (0.26 g, 5.76 mmol), dimethylamine hydrochloride (2.45 g, 30.0 mmol), and sodium cyanoborohydride (1.88 g, 30.0 mmol) in methanol (30 mL) was prepared, 6-Methoxy-1,5-dihydrobenz[cd]indol-4(3H)-one (0.60 g, 3.0 mmol) in methanol (30 mL) was added. The reaction mixture was stirred at ambient temperature for 1 hour, then poured into 5% aqueous sodium bicarbonate solution (100 mL). The resulting mixture was extracted with ethyl acetate (3 × 50 mL) and the combined organic extracts were washed with 5% aqueous hydrochloric acid (3 × 30 mL). The acidic extracts were made basic with 20% aqueous sodium hydroxide and extracted with ethyl acetate (3 × 50 mL), the combined organic extracts were washed with 10% aqueous sodium sulfate, dried and concentrated to yield a product which was purified by flash chromatography using 5% diisopropylamine-ethyl acetate as eluant to give N,N-dimethyl-6-methoxy-1,3,4,5-tetrahydrobenz[cd]indol-4-amine as an oil. This free base was dissolved in methanol (10mL) and treated with a solution of oxalic acid (0.156 g, 1.73 mmol) in methanol (5 mL). The crystalline product was collected and dried (25°C, 0.1 torr) to yield N,N-dimethyl-6-methoxy-1,3,4,5-tetrahydrobenz[cd]indol-4-amine oxalate.

Example 4

By the procedure of Example 3, using in place of dimethylamine the following amines;

dibenzylamine

dicyclohexylamine

aniline

the products are:

N,N-dibenzyl-6-methoxy-1,3,4,5-tetrahydrobenz[cd]indol-4-amine

N,N-dicyclohexyl-6-methoxy-1,3,4,5-tetrahydrobenz[cd]indol-4-amine
N-phenyl-6-methoxy-1,3,4,5-tetrahydrobenz[cd]indol-4-amine.

Similarly, using N-butyl-N-propylamine in place of dimethylamine, the product is N-butyl-N-propyl-6-methoxy-1,3,4,5-tetrahydrobenz[cd]indol-4-amine (only for illustrative purposes, not claimed).

Example 5

A mixture of 6-methoxy-1,3,4,5-tetrahydrobenz[cd]indol-4-amine (2.0 g, 0.01 mol,) benzoyl chloride (2.1 g, 0.015 mol) and 10 ml of pyridine is stirred at room temperature for 10 minutes, then poured into water. Extracting with ethyl acetate and then concentrating the extracts gives N-benzoyl-6-methoxy-1,3,4,5-tetrahydrobenz[cd]indol-4-amine as the residue.

One gram of this N-benzoyl compound in 20 ml of tetrahydrofuran is added to one gram of lithium aluminum hydride in 20 ml of tetrahydrofuran and the resulting mixture is stirred at room temperature overnight, then quenched with water, filtered and concentrated to give N-benzyl-6-methoxy-1,3,4,5-tetrahydrobenz[cd]indol-4-amine.

By the same procedure, using 6-hydroxy-1,3,4,5-tetrahydrobenz[cd]indol-4-amine in place of the 6-methoxy compound, the product is N-benzyl-6-hydroxy-1,3,4,5-tetrahydrobenz[cd]indol-4-amine.

Example 6

A mixture of 6-methoxy-1,3,4,5-tetrahydrobenz[cd]indol-4-amine (2.0 g, 0.01 mol) and p-nitrophenyl formate (1.7 g, 0.01 mol) in 25 ml of ethyl acetate and 10 ml of pyridine is stirred overnight at room temperature, then washed with water and concentrated to give N-formyl-6-methoxy-1,3,4,5-tetrahydrobenz[cd]indol-4-amine.

The above prepared N-formyl compound is reduced with lithium aluminum hydride in tetrahydrofuran and the resulting mixture is worked up by the procedure of Example 5 to give N-methyl-6-methoxy-1,3,4,5-tetrahydrobenz[cd]indol-4-amine.

By the same procedure, using 0.01 mol of acetic anhydride in ethyl acetate, the product is N-acetyl-6-methoxy-1,3,4,5-tetrahydrobenz[cd]indol-4-amine. Reducing with lithium aluminum hydride in tetrahydrofuran by the procedure of Example 5 gives N-ethyl-6-methoxy-1,3,4,5-tetrahydrobenz[cd]indol-4-amine.

Using 6-hydroxy-1,3,4,5-tetrahydrobenz[cd]indol-4-amine in place of the corresponding 6-methoxy compound in the above procedures, the products are:

N-methyl-6-hydroxy-1,3,4,5-tetrahydrobenz[cd]indol-4-amine and N-ethyl-6-hydroxy-1,3,4,5-tetrahydrobenz[cd]indol-4-amine.

Example 7

By the procedure of Example 2, the following 6-methoxy compounds of Example 4 are reacted with boron tribromide:

N,N-dibenzyl-6-methoxy-1,3,4,5-tetrahydrobenz[cd]indol-4-amine
N-phenyl-6-methoxy-1,3,4,5-tetrahydrobenz[cd]indol-4-amine

to give the corresponding 6-hydroxy compounds.

Example 8

6-Methoxy-1,3,4,5-tetrahydrobenz[cd]indol-4-amine (50 mg) is mixed with 100 mg of lactose and 3 mg of magnesium stearate. The resulting mixture is filled into a hard gelatin capsule.

**Claims for the Contracting States: BE CH DE FR GB IT LI LU NL SE**

1. A compound of formula (I):

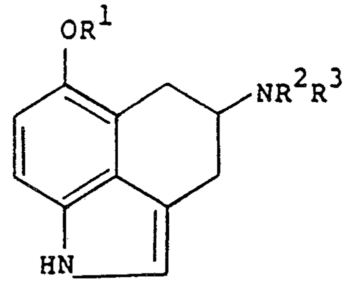

(I)

in which:

$R^1$ is hydrogen or $C_{1-5}$ alkyl; and

$R^2$ and $R^3$, being the same or different, are hydrogen, $C_{1-4}$ alkyl, phenyl$(CH_2)_n$ where n is 0—3, $C_{5-6}$ cycloalkyl or $C_{3-5}$ alkenyl;

or a pharmaceutically acceptable acid addition salt thereof, provided that, when

i) $R^1$ is $C_{1-4}$alkyl, $R^2$ and $R^3$ are not both $C_{1-4}$alkyl or allyl, or pharmaceutically acceptable salts thereof except the oxalate salt of the compound in which $R^1$ to $R^3$ are all methyl;

ii) $R^1$ is hydrogen, $R^2$ and $R^3$ are not both $C_{1-4}$alkyl or allyl, of pharmaceutically acceptable salts thereof.

2. A compound according to claim 1 in which $R^1$ is hydrogen or methyl.

3. A compound according to claim 2, which is 6-methoxy-1,3,4,5-tetrahydrobenz[cd]indol-4-amine; or N,N-dimethyl-6-methoxy-1,3,4,5-tetrahydrobenz[cd]indol-4-amine oxalate.

4. A pharmaceutical composition comprising a compound of formula (I) or a pharmaceutically acceptable acid addition salt thereof as claimed in claim 1 in association with a pharmaceutically acceptable carrier.

5. A compound of formula (I) or a pharmaceutically acceptable salt thereof as claimed in claim 1 for use as a therapeutic agent.

6. A compound of formula (I) or a pharmaceutically acceptable salt thereof as claimed in claim 1 for use as 5—$HT_1$ receptor antagonists.

7. The use of a compound of formula (I) or a pharmaceutically acceptable salt thereof as claimed in claim 1 for the manufacture of an antihypertensive composition.

8. A process for the preparation of a compound of formula (I) as claimed in claim 1 which comprises:

(a) reductive amination of a compound of formula (III):

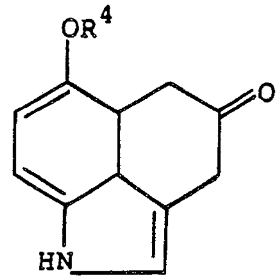

(III)

in which $R^4$ is $C_{1-5}$ alkyl, with sodium cyanoborohydride and an amine of the formula:

$R^2R^3NH$

in which $R^2$ and $R^3$ are as defined in claim 1, and an inorganic salt of the amine, to form a compound of formula (I) in which $R^1$ is $C_{1-5}$ alkyl, and optionally, where desired, dealkylating the group $R^1$ to form a compound of formula (I) in which $R^1$ is hydrogen and, optionally, forming a pharmaceutically acceptable acid addition salt thereof; or

(b) hydrogenation of a compound of formula (II):

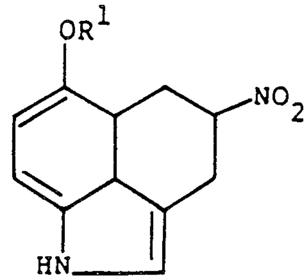

(II)

in which $R^1$ is hydrogen or $C_{1-5}$ alkyl, to form a compound of formula (I) wherein $R^2$ and $R^3$ are both hydrogen, and, optionally, forming a pharmaceutically acceptable acid addition salt thereof.

9. A process as claimed in claim 8(a) in which the inorganic acid addition salt of the amine and the basic amine are present in a ratio of about 10:1 to 10:3.

10. A compound of the formula:

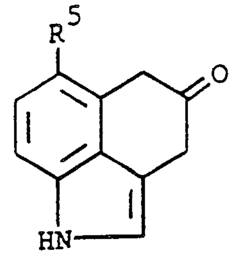

(IV)

in which $R^5$ is $C_{1-5}$ alkoxy.

11. A compound according to claim 10, which is 6-methoxy-1,5-dihydrobenz[cd]indol-4(3H)-one.

**Claims for the Contracting State: AT**

1. A process for preparing a compound of formula (I):

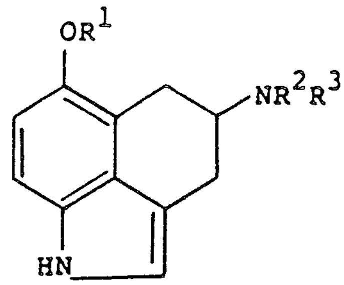

(I)

in which:

$R^1$ is hydrogen or $C_{1-5}$ alkyl; and

$R^2$ and $R^3$, being the same or different, are hydrogen, $C_{1-4}$ alkyl, phenyl$(CH_2)_n$ where n is 0—3, $C_{5-6}$ cycloalkyl or $C_{3-5}$ alkenyl;

or a pharmaceutically acceptable acid addition salt thereof, provided that, when

i) $R^1$ is $C_{1-4}$alkyl, $R^2$ and $R^3$ are not both $C_{1-4}$alkyl or allyl, or pharmaceutically acceptable salts thereof except the oxalate salt of the compound in which $R^1$ to $R^3$ are all methyl;

ii) $R^1$ is hydrogen, $R^2$ and $R^3$ are not both $C_{1-4}$alkyl or allyl, of pharmaceutically acceptable salts thereof, which comprises hydrogenating a compound of the formula:

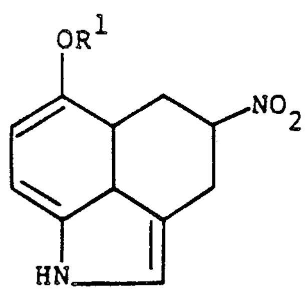

(II)

in which $R^1$ is as defined above and, optionally where desired forming a pharmaceutically acceptable acid addition salt thereof.

2. A process according to claim 1 in which 6-methoxy-1,3,4,5-tetrahydrobenz[cd]indol-4-amine is prepared by hydrogenating 6-methoxy-4-nitro-1,3,4,5-tetrahydrobenz[cd]indole using a noble metal catalyst.

3. A process for preparing a compound of formula (I) as defined in claim 1 by reductive amination of a compound of the formula:

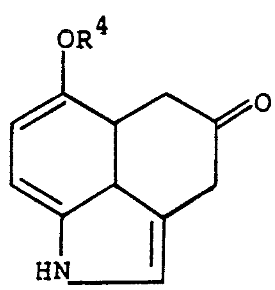

(III)

in which $R^4$ is $C_{1-5}$ alkyl using sodium cyanoborohydride and an amine of the formula:

$$R^2R^3NH$$

in which $R^2$ and $R^3$ are as defined above, and an inorganic acid addition salt of said amine and, to form a compound of formula (I) in which $R^1$ is $C_{1-5}$ alkyl, and, optionally where desired forming a pharmaceutically acceptable acid addition salt thereof.

4. A process according to claim 3 in which the inorganic acid addition salt of the amine and the basic amine are present in a ratio of about 10:1 to 10:3.

5. A process for preparing a pharmaceutical composition which comprises bringing into association a compound of formula (I) or a pharmaceutically acceptable acid addition salt thereof as defined in claim 1 and a pharmaceutical carrier.

6. A process as claimed in claim 5 in which the compound of formula (I) is 6-methoxy-1,3,4,5-tetrahydrobenz[cd]indol-4-amine; or N,N-dimethyl-6-methoxy-1,3,4,5-tetrahydrobenz[cd]indol-4-amine oxalate.

7. A process for the preparation of a compound of formula (III):

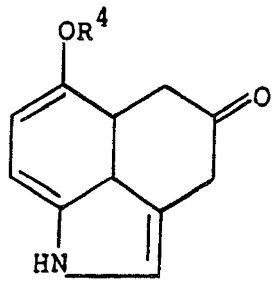

(III)

in which $R^4$ is $C_{1-5}$ alkyl which comprises reaction of a compound of formula:

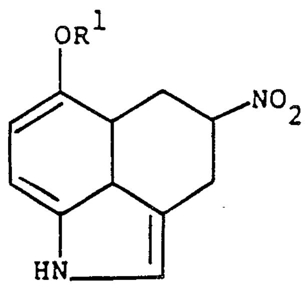

(II)

in which $R^1$ is $C_{1-5}$ alkyl with sodium methoxide in methanol followed by aqueous titantium tetrachloride.

**Patentansprüche für die Vertragsstaaten: BE CH DE FR GB IT LI LU NL SE**

1. Eine Verbindung der Formel (I)

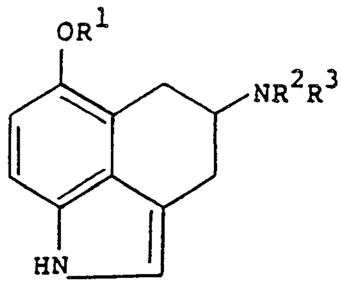

(I)

in der:

$R^1$ ein Wasserstoffatom oder einen $C_{1-5}$-Alkylrest bedeutet; und

$R^2$ und $R^3$, die gleich oder verschieden sind, Wasserstoffatome, $C_{1-4}$-Alkylreste, Phenyl-$(CH_2)_n$, wobei n 0 bis 3 ist, $C_{5-6}$-Cycloalkyl- oder $C_{3-5}$-Alkenylreste darstellen; oder ein pharmazeutisch verträgliches Säureadditionssalz davon, mit der Maßgabe, daß, wenn

i) $R^1$ einen $C_{1-4}$-Alkylrest bedeutet, $R^2$ und $R^3$ nicht beide $C_{1-4}$-Alkyl- oder Allylreste sind, oder pharmazeutisch verträgliche Salze davon mit Ausnahme des Oxalatsalzes der Verbindung, in der $R^1$ bis $R^3$ alle Methylgruppen sind;

ii) $R^1$ ein Wasserstoffatom bedeutet, $R^2$ und $R^3$ nicht beide $C_{1-4}$-Alkyl- oder Allylreste oder pharmazeutisch verträgliche Salze davon bedeuten.

2. Verbindung nach Anspruch 1, in der $R^1$ ein Wasserstoffatom oder eine Methylgruppe ist.

3. Verbindung nach Anspruch 2, nämlich 6-Methoxy-1,3,4,5-tetrahydrobenz[cd]indol-4-amin oder N,N-Dimethyl-6-methoxy-1,3,4,5-tetrahydrobenz[cd]indol-4-amin-oxalat.

4. Arzneimittel, umfassend eine Verbindung der Formel (I) oder ein pharmazeutisch verträgliches Säureadditionssalz davon nach Anspruch 1 in Verbindung mit einem pharmazeutisch verträglichen Träger.

5. Verbindung der Formel (I) oder ein pharmazeutisch verträgliches Salz davon nach Anspruch 1 zur Verwendung als therapeutischer Wirkstoff.

6. Verbindung der Formel (I) oder ein pharmazeutisch verträgliches Salz davon nach Anspruch 1 zur Verwendung als $5HT_1$-Rezeptorantagonisten.

7. Verwendung einer Verbindung der Formel (I) oder ein pharmazeutisch verträgliches Salz davon nach Anspruch 1 zur Herstellung eines blutdrucksenkenden Mittels.

8. Verfahren zur Herstellung einer Verbindung der Formel (I) nach Anspruch 1, welches umfaßt:

(a) reduktive Aminierung einer Verbindung der Formel (III)

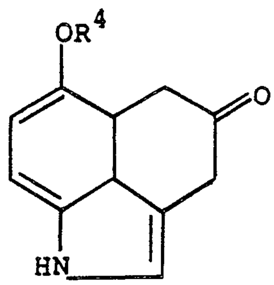

(III)

in der $R^4$ ein $C_{1-5}$-Alkylrest bedeutet, mit Natriumcyanoborhydride und einem Amin der Formel

$$R^2R^3NH$$

in der $R^2$ und $R^3$ wie in Anspruch 1 definiert sind, une einem anorganischen Salz des Amins, zur Erzeugung einer Verbindung der Formel (I), in der $R^1$ einen $C_{1-5}$-Alkylrest bedeutet, und gegebenenfalls, wenn gewünscht, Dealkylierung des Restes $R^1$ zur Herstellung einer Verbindung der Formel (I), in der $R^1$ ein Wasserstoffatom bedeutet, und gegebenenfalls Herstellung eines pharmazeutisch verträglichen Säureadditionssalzes davon; oder

(b) Hydrierung einer Verbindung der Formel (II)

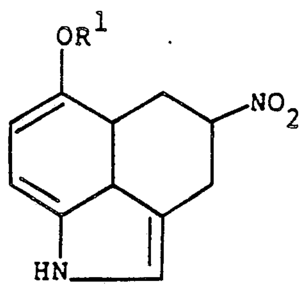

(II)

in der $R^1$ ein Wasserstoffatom oder einen $C_{1-5}$-Alkylrest bedeutet, zur Herstellung einer Verbindung der Formel (I), in der $R^2$ und $R^3$ beide Wasserstoffatome bedeuten, und gegebenenfalls Herstellung eines pharmazeutisch verträglichen Säureadditionssalzes davon.

9. Verfahren nach Anspruch 8(a), wobei das anorganische Säureadditionssalz des Amins und das basische Amin in einem Verhältnis von etwa 10:1 bis 10:3 vorhanden sind.

10. Verbindung der Formel

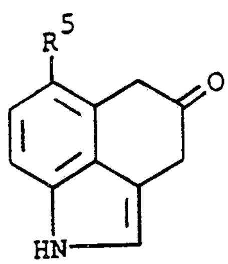

(IV)

in der $R^5$ einen $C_{1-5}$-Alkoxyrest bedeutet.

11. Verbindung nach Anspruch 10, nämlich 6-Methoxy-1-5-dihydrobenz[cd]indol-4-(3H)-on.

**Patentansprüche für den Vertragsstaat: AT**

1. Verfahren zur Herstellung einer Verbindung der Formel (I)

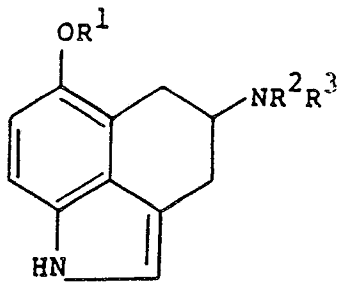

(I)

in der:

$R^1$ ein Wasserstoffatom oder einen $C_{1-5}$-Alkylrest bedeutet; und
$R^2$ und $R^3$, die gleich oder verschieden sind, Wasserstoffatome, $C_{1-4}$-Alkylreste, Phenyl-$(CH_2)_n$, wobei n 0 bis 3 ist, $C_{5-6}$-Cycloalkyl- oder $C_{3-5}$-Alkenylreste darstellen; oder ein pharmazeutisch verträgliches Säureadditionssalz davon, mit der Maßgabe, daß, wenn
i) $R^1$ einen $C_{1-4}$-Alkylrest bedeutet, $R^2$ und $R^3$ nicht beide $C_{1-4}$-Alkyl- oder Allylreste sind, oder pharmazeutisch verträgliche Salze davon mit Ausnahme des Oxalatsalzes der Verbindung, in der $R^1$ bis $R^3$ alle Methylgruppen sind;
ii) $R^1$ ein Wasserstoffatom bedeutet, $R^2$ und $R^3$ nicht beide $C_{1-4}$-Alkyl- oder Allylreste oder pharmazeutisch verträgliche Salze davon bedeuten,
welches umfaßt die Hydrierung einer Verbindung der Formel (II)

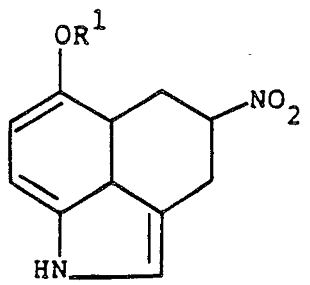

(II)

in der $R^1$ wie vorstehend definiert ist, und gegebenenfalls, wenn gewünscht, eines pharmazeutisch verträglichen Säureadditionssalzes davon.

2. Verfahren nach Anspruch 1, wobei 6-Methoxy-1,3,4,5-tetrahydrobenz[cd]indol-4-amin durch Hydrierung von 6-Methoxy-4-Nitro-1,3,4,5-tetrahydrobenz[cd]indol unter Verwendung eines Edelmetallkatalysators hergestellt wird.

3. Verfahren zur Herstellung einer Verbindung der Formel (I) nach Anspruch 1 durch reduktive Aminierung einer Verbindung der Formel

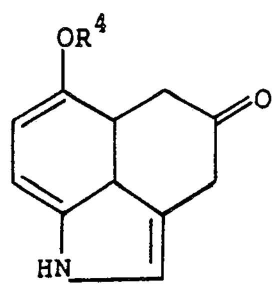

(III)

in der $R^4$ einen $C_{1-5}$-Alkylrest bedeutet, unter Verwendung von Natriumcyanoborhydrid und eines Amins der Formel

$$R^2R^3NH$$

in der $R^2$ und $R^3$ wie vorstehende definiert sind, und eines anorganischen Säureadditionssalzes des Amins, zur Erzeugung einer Verbindung der Formel (I), in der $R^1$ einen $C_{1-5}$-Alkylrest bedeutet, und,

gegebenenfalls, wenn gewünscht, Herstellung eines pharmazeutisch verträglichen Säureadditionssalzes davon.

4. Verfahren nach Anspruch 3, wobei das anorganische Säureadditionssalz des Amins und das basische Amin in einem Verhältnis von etwa 10:1 bis 10:3 vorhanden sind.

5. Verfahren zur Herstellung eines Arzneimittels, welches Zusammenbringen einer Verbindung der Formel (I) oder eines pharmazeutisch verträglichen Säureadditionssalzes davon gemäß Anspruch 1 und eines pharmazeutischen Trägers umfaßt.

6. Verfahren nach Anspruch 5, wobei die Verbindung der Formel (I) 6-Methoxy-1,3,4,5-tetrahydrobenz[cd]indol-4-amin oder N,N-Dimethyl-6-methoxy-1,3,4,5-tetrahydrobenz[cd]indol-4-amin-oxalat ist.

7. Verfahren zur Herstellung einer Verbindung der Formel (III)

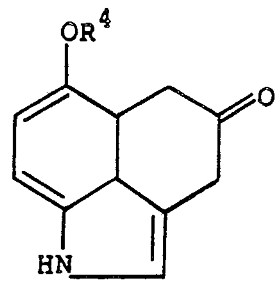

(III)

in der $R^4$ einen $C_{1-5}$-Alkylrest bedeutet, welches Umsetzung einer Verbindung der Formel

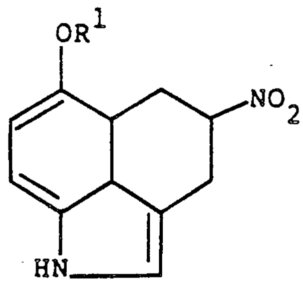

(II)

in der $R^1$ einen $C_{1-5}$-Alkylrest darstellt, mit Natriummethoxid in Methanol, gefolgt von wäßrigem Titantetrachlorid, umfaßt.

**Revendications pour lest Etats contractants: BE CH DE FR GB IT LI LU NL SE**

1. Un composé de formule (I):

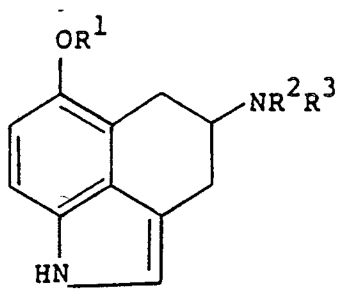

(I)

dans laquelle:

$R^1$ est de l'hydrogène ou un alkyle en $C_{1-5}$; et

$R^2$ et $R^3$ qui sont identiques ou différents sont de l'hydrogène, un alkyle en $C_{1-4}$, un phényl$(CH_2)_n$ où n est 0—3, un cycloalkyle en $C_{5-6}$ ou un alcényle en $C_{3-5}$;

ou un de ses sels d'addition acide pharmaceutiquement acceptables à condition que, lorsque

i) $R^1$ est un alkyle en $C_{1-4}$, $R^2$ et $R^3$ ne soient pas tous deux un alkyle en $C_{1-4}$ ou allyle ou leurs sels pharmaceutiquement acceptables à l'exception de l'oxalate du composé dans lequel $R^1$ à $R^3$ sont tous méthyle;

ii) $R^1$ est de l'hydrogène, $R^2$ et $R^3$ ne soient pas tous deux un alkyle en $C_{1-4}$ ou allyle ou leurs sels pharmaceutiquement acceptables.

2. Composé suivant la revendication 1 dans laquelle $R^1$ est de l'hydrogène ou méthyle.

3. Composé suivant la revendication 2 qui est la 6-méthoxy-1,3,4,5-tétrahydrobenz[cd]indol-4-amine ou l'oxalate de N,N-diméthyl-6-méthoxy-1,3,4,5-tétrahydrobenz[cd]indol-4-amine.

4. Composition pharmaceutique comprenant un composé de formule (I) ou un de ses sels d'addition acide pharmaceutiquement acceptables tel que revendiqué dans la revendication 1 en association avec un support pharmaceutiquement acceptable.

5. Composé de formule (I) ou sel pharmaceutiquement acceptable de celui-ci tel que revendiqué dans la revendication 1 pour usage comme agent thérapeutique.

6. Composé de formule (I) ou sel pharmaceutiquement acceptable de celui-ci tel que revendiqué dans la revendication 1, pour usage comme antagoniste au niveau de récepteur $5HT_1$.

7. Utilisation d'un composé de formule (I) ou d'un sel pharmaceutiquement acceptable de celui-ci tel que revendiqué dans la revendication 1 pour la fabrication d'une composition antihypertensive.

8. Procédé pour la préparation d'un composé de formule (I) tel que revendiqué dans la revendication 1 qui comprend:

(a) l'amination réductrice d'un composé de formule (III):

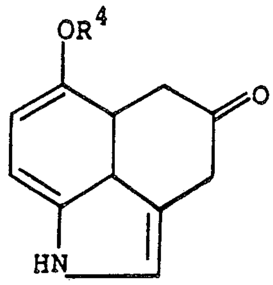

(III)

dans laquelle $R^4$ est un alkyle en $C_{1-5}$ avec du cyanoborohydrure de sodium et une amine de formule:

$$R^2R^3NH$$

dans laquelle $R^2$ et $R^3$ sont tels que définis dans la revendication 1 et un sel inorganique de l'amine pour former un composé de formule (I) dans laquelle $R^1$ est un alkyle en $C_{1-5}$ et, facultativement, si on le désire, la déalkylation du groupement $R^1$ pour former un composé de formule (I) dans laquelle $R^1$ est de l'hydrogène et, facultativement, la formation d'un sel d'addition acide pharmaceutiquement acceptable de celui-ci; ou

(b) l'hydrogénation d'un composé de formule (II):

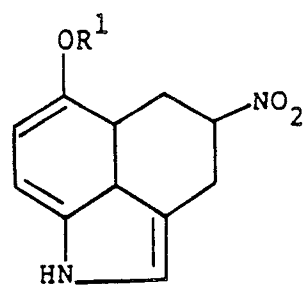

(II)

dans laquelle $R^1$ est de l'hydrogène ou un alkyle en $C_{1-5}$ pour former un composé de formule (I) dans laquelle $R^2$ et $R^3$ sont tous deux de l'hydrogène et, facultativement, la formation d'un sel d'addition acide pharmaceutiquement acceptable de celui-ci.

9. Procédé tel que revendiqué dans la revendication 8(a) dans laquelle le sel d'addition acide inorganique de l'amine et l'amine basique sont présents dans un rapport d'environ 10:1 à 10:3.

10. Composé de formule:

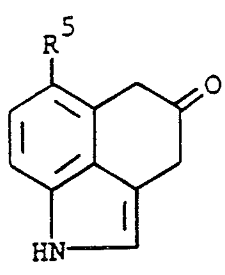

(IV)

dans laquelle $R^5$ est un alkoxy en $C_{1-5}$.

11. Composé suivant la revendication 10 qui est la 6-méthoxy-1,5-dihydrobenz[cd]indol-4(3H)-one.

**Revendication pour l'Etat contractant: AT**

1. Procédé pour préparer un composé de formule (I):

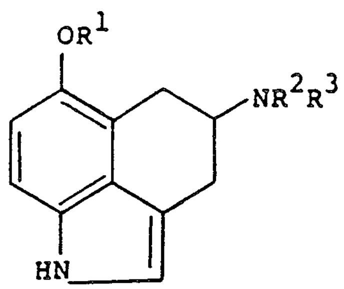

(I)

dans laquelle:

$R^1$ est de l'hydrogène ou un alkyle en $C_{1-5}$; et

$R^2$ et $R^3$ qui sont identiques ou différents sont de l'hydrogène, un alkyle en $C_{1-4}$, un phényl$(CH_2)_n$ où n est 0—3, un cycloalkyle en $C_{5-6}$ ou un alcényle en $C_{3-5}$;

ou un de ses sels d'addition pharmaceutiquement acceptables à la condition que, lorsque

i) $R^1$ est un alkyle en $C_{1-4}$, $R^2$ et $R^3$ ne soient pas tous deux un alkyle en $C_{1-4}$ ou allyle ou leurs sels pharmaceutiquement acceptables à l'exception de l'oxalate du composé dans lequel $R^1$ à $R^3$ sont tous méthyle;

ii) $R^1$ est de l'hydrogène, $R^2$ et $R^3$ ne soient pas tous deux un alkyle en $C_{1-4}$ ou allyle ou leurs sels pharmaceutiquement acceptables,

qui comprend l'hydrogenation d'un composé de formule:

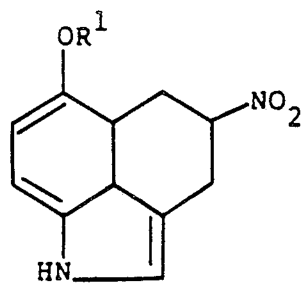

(II)

dans laquelle $R^1$ est tel que défini plus haut et facultativement si on le désire la formation d'un de ses sels d'addition acide pharmaceutiquement acceptables.

2. Procédé suivant la revendication 1 dans laquelle la 6-méthoxy-1,3,4,5-tétrahydrobenz[cd]indol-4-amine est préparée par hydrogénation du 6-méthoxy-4-nitro-1,3,4,5-tétrahydrobenz[cd]-indole en utilisant un métal noble comme catalyseur.

3. Procédé pour préparer un composé de formule (I) tel que défini dans la revendication 1 par amination réductrice d'un composé de formule:

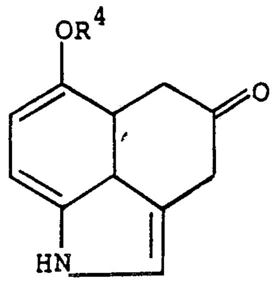

(III)

dans laquelle $R^4$ est un alkyle en $C_{1-5}$ en utilisant du cyanoborohydrure de sodium et une amine de formule:

$$R^2R^3NH$$

dans laquelle $R^2$ et $R^3$ sont tels que définis plus haut et un sel d'addition acide inorganique de ladite amine pour former un composé de formule (I) dans laquelle $R^1$ est un alkyle en $C_{1-5}$ et, facultativement si on le désire, la formation d'un de ses sels d'addition acide pharmaceutiquement acceptable.

4. Procédé suivant la revendication 3 dans laquelle le sel d'addition acide inorganique de l'amine et l'amine basique sont preséńts dans un rapport d'environ 10:1 à 10:3.

5. Procédé pour préparer une composition pharmaceutique qui comprend la mise en association d'un composé de formule (I) ou d'un de ses sels d'addition acide pharmaceutiquement acceptables tel que défini dans la revendication 1 avec un support pharmaceutique.

6. Procédé tel que revendiqué dans la revendication 5 dans laquelle le composé de formule (I) est la 6-méthoxy-1,3,4,5-tétrahydrobenz[cd]indole-4-amine ou l'oxalate de N,N-diméthyl-6-méthoxy-1,3,4,5-tétrahydrobenz[cd]indol-4-amine.

7. Procédé pour la préparation d'un composé de formule (III):

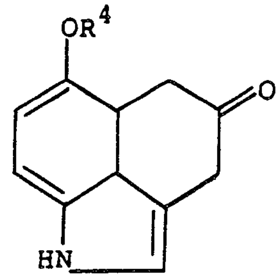

(III)

dans laquelle $R^4$ est un alkyle en $C_{1-5}$ qui comprend la réaction d'un composé de formule:

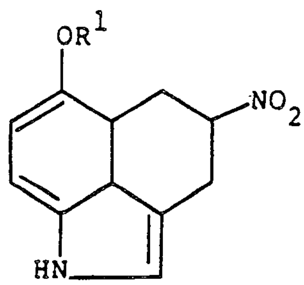

(II)

dans laquelle $R^1$ est un alkyle en $C_{1-5}$ avec du méthylate de sodium dans du méthanol suivie d'un traitement avec du tétrachlorure de titane aqueux.